# EUROPEAN PATENT APPLICATION

(11) **EP 3 771 465 A1**
(43) Date of publication of application: **03.02.2021**
(21) Application number: 19460039.1
(22) Date of filing: 01.08.2019
(51) Int. Cl.: A61K 31/4439, A61P 7/02, A61P 9/10, A61K 9/50

(54) **PHARMACEUTICAL COMPOSITION COMPRISING DABIGATRAN ETEXILATE**

(71) Applicant: Zaklady Farmaceutyczne Polpharma SA, 83-200 Starogard Gdanski (PL)
(72) Inventor: Hrakovsky, Julia, 80-862 Gdansk (PL); Przerada, Szymon, 83-200 Starogard Gdanski (PL); Kaczmarek, Mateusz, 63-400 Ostrów Wielkopolski (PL); Skoczen, Przemyslaw, 83-010 Straszyn (PL)
(74) Representative: Obrycki, Pawel Andrzej

(57) **Abstract**

The present invention is related to a pharmaceutical composition for oral administration comprising a core comprising a pharmaceutically acceptable organic acid; optionally an isolating layer comprising a pharmaceutically acceptable polymer; and an active substance layer comprising dabigatran etexilate or a pharmaceutically acceptable salt thereof and a binder; wherein weight ratio between the pharmaceutically acceptable organic acid and the active substance is comprised between 0.15:1 and 0.40:1, and wherein the weight of active substance in said ratio is expressed as equivalent weight of dabigatran etexilate mesylate. The invention also relates to a method of preparation of this pharmaceutical composition and its use in the treatment and/or prevention of blood coagulation, thromboembolic events and stroke.

## Description

### Technical field

The present invention relates to pharmaceutical compositions comprising dabigatran etexilate, or a pharmaceutically acceptable salt thereof, as active ingredient.

### State of the art

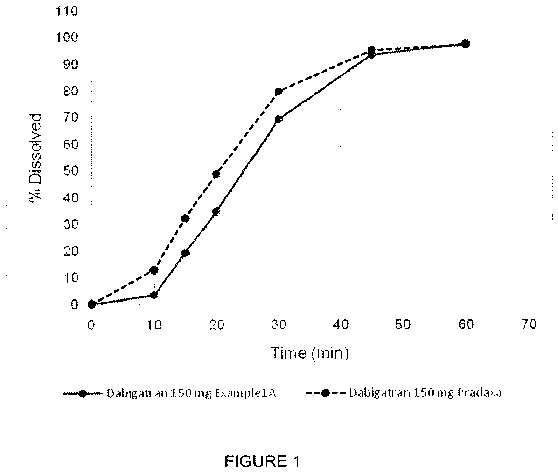

Dabigatran etexilate is an anticoagulant drug which is commercially available under the trade name Pradaxa®. The chemical structure of dabigatran etexilate is shown in Figure 1.

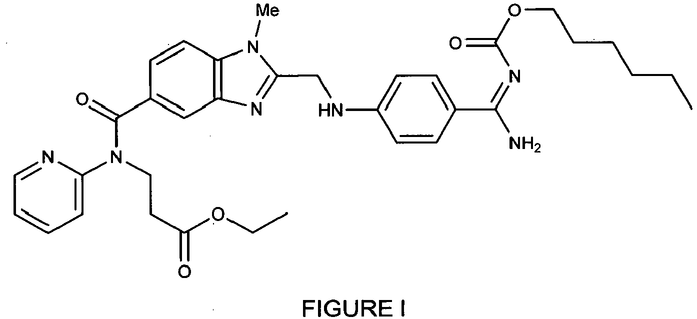

Dabigatran etexilate is a basic substance, and is commercially available in the form of a pharmaceutically acceptable salt, namely, it is available as the salt with methanesulfonic acid (mesylate salt).

The dabigatran etexilate molecule is in fact a double prodrug of the active moiety dabigatran (Figure II). After oral administration, dabigatran etexilate is rapidly absorbed and converted to the active compound dabigatran by esterase-catalysed hydrolysis in plasma and in the liver. This is a two-step process involving hydrolysis of an ethyl ester and a carbamate ester producing the active moiety,

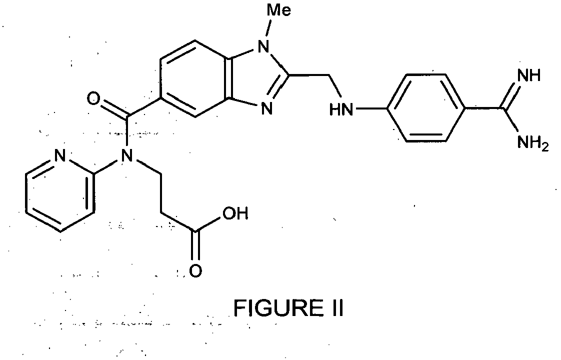

Dabigatran is a potent, competitive, reversible, direct thrombin inhibitor. Since thrombin enables the conversion of fibrinogen into fibrin during the coagulation cascade, its inhibition prevents the development of thrombus. The main indications of dabigatran are the prevention venous thromboembolic events, such as deep vein thrombosis, pulmonary embolism or systemic embolism, and the prevention of stroke.

Dabigatran etexilate has low solubility in water and high intrinsic passive permeability, and is considered a Class II compound according to the biopharmaceutical classification system, so dissolution is most likely the rate-limiting step for drug absorption. Solubility is strongly pH dependent, with increased solubility at acidic pH.

On the other hand, dabigatran etexilate is susceptible to hydrolysis in presence of humidity and under acidic conditions.

Pradaxa® is in the form of pellets contained in hard hydroxypropyl methylcellulose (HPMC) capsules, and is available in the 75, 110, and 150 mg strengths, expressed as the amount of dabigatran etexilate free base. Said pellet formulation contains about 40 wt% of tartaric acid. The function of tartaric acid in the formulation is to provide an acid microenvironment after dissolution, in order to maximise the dissolution and the absorption of the active ingredient. On the other hand, due to the known hydrolysis susceptibility of dabigatran etexilate, the formulation is made of multi-layered pellets, where the tartaric acid and the active ingredient are not in direct contact.

In this regard, the low aqueous solubility of dabigatran etexilate and its instability in the presence of acids and humidity has influenced the development of stable and bioavailable oral pharmaceutical dosage forms.

Several dabigatran etexilate formulations have been proposed so far in the art.

Thus, for example, the composition and manufacturing method of Pradaxa® are disclosed in the international patent applications WO03/074056-A1, WO2009/118321-A1, WO2009/118322-A1 and WO2010/007016-A1.

In the patent application WO03/074056-A1, it is disclosed the use of pharmaceutically acceptable organic acids, preferably, tartaric, fumaric, succinic or citric acid, in the formulation of dabigatran etexilate, or its pharmaceutically acceptable salts, namely, the mesylate salt. The formulations are in the form of multi-layered pellets, comprising a core material containing the acid, an isolating layer over the core, and an active substance layer over the isolating layer. The isolating layer comprises a water-soluble polymer, such as acacia gum or HPMC. The active substance layer contains dabigatran etexilate, or a pharmaceutically acceptable salt thereof, and a binding agent, such as hydroxypropyl cellulose (HPC). Both layers may also include an anticaking agent, such as talc. Optionally, the pellets may have an additional outermost finishing layer. It is stated that the preferred ratio acid:active substance is from about 0.9:1 to about 4:1, preferably from about 1:1 to about 3:1, and the upper limit (4:1) is generally determined by the maximum acceptable size of the preparation in the desired dosages (number of pellets per capsule).

In the patent application WO2009/118321-A1 a powder layering process for preparing the tartaric cores is disclosed, which ensures better uniformity, and sphere-like geometry. The method comprises first coating tartaric acid particles having particle size in the range 0.2-0.8 mm with a solution of tartaric acid and acacia gum, then sprinkling the coated particles with fine tartaric acid powder, and alternately repeating both steps until forming the cores. The tartaric acid cores obtained are subsequently covered with an isolating layer by spraying a suspension of HPMC, dimethicone and talc in ethanol. The insulated tartaric pellets obtained by this process may be used for the preparations containing dabigatran as described in the examples of WO03/074056-A1.

In the patent applications WO2009/118322-A1 and WO2010/007016-A1 the tartaric acid cores are analogously prepared by powder layering and covered with an isolating layer. A dabigatran etexilate mesylate suspension is prepared and sprayed onto these isolated cores in one or more process steps. Said suspension contains dabigatran etexilate mesylate, HPC and talc in isopropanol, and is prepared maintaining the temperature under 30° C to ensure process reproducibility and to avoid a change in the polymorphic form of the active substance. The ratio tartaric acid:active substance in the examples is about 1:1.

Other alternative formulations are disclosed in later documents of the state of the art.

For example, in the international patent application WO2012/001156-A2, a process for preparing pellets of dabigatran etexilate, or a pharmaceutically acceptable salt thereof, is disclosed wherein the tartaric acid core is prepared using spherical commercially available inert pellets as starting material, for example, sucrose, microcrystalline cellulose, starch or tartaric acid pellets, which are then coated with a solution of tartaric acid and a binder. Said tartaric acid core is coated with an isolating layer, with a layer containing the active ingredient and, optionally, with an overcoat. The ratio tartaric acid:active substance in the pellets disclosed in the examples is about 0.7:1 and about 1:1.

The international patent application WO2012/162492-A1 discloses dabigatran etexilate mesylate formulations in the form of capsules containing several mini-tablets, instead of pellets. Thus, for example, a 75 mg strength capsule contains 12 mini-tablets (Example 9). Each mini-tablet comprises a compressed core made of tartaric acid, microcrystalline cellulose and magnesium stearate; said core is coated with a first layer comprising HPMC, talc and polyethylene glycol 400; and with a second drug layer comprising dabigatran etexilate mesylate, HPC and talc. The ratio tartaric acid:active substance in the formulation is about 0.9:1.

The international patent application WO2018/229784-A1 also discloses a dabigatran etexilate, or a pharmaceutically acceptable salt thereof, pharmaceutical composition in the form of multi-layered pellets, comprising a core comprising tartaric acid, an isolating layer coated over the core and an active substance layer over the isolating layer comprising the active ingredient and binder in an amount of 13 to 40 wt%. In the example provided, the ratio between tartaric acid and active substance is about 0.9:1.

The dabigatran etexilate formulations described so far in the art comprising an organic acid in the formulation, particularly tartaric acid, have proved to be effective to provide an acidic microenvironment when the formulation is dissolved, thus ensuring an optimal bioavailability of dabigatran etexilate. However, relatively high amount of acid needs to be used in the formulations disclosed in the prior art, which can give rise to potential side effects due to the erosive effect of the acid.

Therefore, there is the need to provide improved dabigatran etexilate formulations which are stable, which have good solubility and which are able to provide good bioavailability of the active agent, while minimizing any possible adverse effects.

### Object of the invention

The object of the present invention is a pharmaceutical composition comprising dabigatran or a pharmaceutically acceptable salt thereof.

Another aspect of the invention is a pharmaceutical dosage form comprising said pharmaceutical composition.

Another aspect of the invention is a process for the preparation of such pharmaceutical composition.

Another aspect of the invention is the pharmaceutical composition for use in therapy, particularly, for the treatment and/or prevention of blood coagulation, thromboembolic events and stroke.

### Description of the Drawings

Figure 1 represents the results of a comparative *in vitro* dissolution assay, as disclosed in Example 2, of a pharmaceutical composition according to the present invention (solid line) and the commercial product Pradaxa® (dotted line).

### Detailed description of the invention

The object of the present invention is a pharmaceutical composition for oral administration comprising:
a) a core comprising a pharmaceutically acceptable organic acid;
b) optionally, an isolating layer comprising a pharmaceutically acceptable polymer; and
c) an active substance layer comprising dabigatran etexilate or a pharmaceutically acceptable salt thereof, preferably dabigatran etexilate mesylate, and a binder;
wherein weight ratio between the pharmaceutically acceptable organic acid and the active substance is comprised between 0.15:1 and 0.40:1, and wherein the weight of the active substance in said ratio is expressed as equivalent weight of dabigatran etexilate mesylate.

The authors of the present invention have developed a pharmaceutical composition for the oral administration of dabigatran etexilate or a pharmaceutically acceptable salt thereof in the form of a multi-layered composition, wherein the required properties or stability and solubility of the composition as well as the bioavailability of the active substance are achieved using significantly lower amount of tartaric acid.

Indeed, surprisingly, the developed formulation was bioequivalent to Pradaxa® despite comprising about 60-80% less organic acid (tartaric acid).

Therefore, according to the present invention, significantly lower amount of excipients are contained in each dosage form, thus reducing potential allergy or even hypersensitivity for some of them. The risk of undesirable side effects is reduced and, particularly, the risk of gastro intestinal adverse reactions caused by erosive effect of the acid.

Another benefit of reducing the amount of excipients is that the volume of the final dosage form can be reduced and can facilitate its swallowing and, therefore, can also prompt improved patient compliance.

Indeed, as shown in Example 1A, capsules filled with the pharmaceutical composition according to the invention contained 146 mg, 214 mg and 292 mg of the composition for the 75 mg, 110 mg and 150 mg dabigatran etexilate strengths, respectively, which means about 30% weight reduction of Pradaxa® composition for equivalent strengths, as shown in Table I:

**TABLE I**

| *Strength* | *Tartaric acid: dabigatran etexilate mesylate ratio* | *Filling weight (mg*/*capsule)* | | |
|---|---|---|---|---|
| | | *75 mg* | *110 mg* | *150 mg* |
| Pradaxa® | 1:1 | 209 | 311 | 415 |
| Example 1A | 0.30:1 | 146 | 214 | 292 |

Pradaxa® capsules sizes are "0", "1" and "2" for strengths 150 mg, 110 mg and 75 mg, respectively.

The present invention would allow using smaller size capsules, for example, "1", "2" and "3" for equivalent dabigatran etexilate strengths.

Along the present description, as well as in the claims, the singular expressions, generally preceded by the articles "a", "an" or "the", are meant to include also the plural forms, unless the context clearly indicates otherwise. Furthermore, numeric values preceded by the term "about" are meant to include the exact stated value and also a certain variation around such value, namely a variation or ±5% of the stated amount. Numeric ranges defined by lower and upper endpoints are meant to include also said stated endpoints. The percentages disclosed (%, wt%) are always weight percentages.

### Dabigatran

Dabigatran etexilate is the International Nonproprietary Name (INN) assigned to the substance ethyl 3-[[[2-[[[4-[[[(hexyloxy)carbonyl]amino]iminomethyl]phenyl]amino] methyl]-1-methyl-1*H*-benzimidazol-5-yl]carbonyl](pyridin-2-yl)amino]propanoate, whose empirical chemical formula is C₃₄H₄₁N₇O₅ and can be represented as follows:

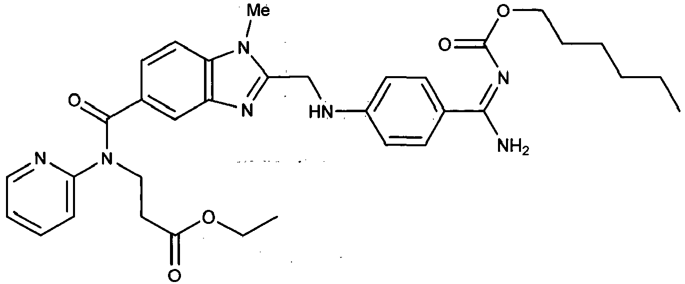

Dabigatran etexilate is an orally active anticoagulant. Dabigatran etexilate molecule is in fact a prodrug which is absorbed after oral administration and rapidly converted to dabigatran by esterase-catalysed hydrolysis in plasma and in the liver. Dabigatran is a direct thrombin inhibitor and therefore can be therapeutically used, for example, in the treatment and prevention of blood coagulation, thromboembolic events and stroke.

The pharmaceutical compositions of the present invention contain dabigatran etexilate or a pharmaceutically acceptable salt thereof as active substance. Said pharmaceutically acceptable salts of dabigatran etexilate are addition salts with pharmaceutically acceptable acids, for example, with methanesulfonic, hydrochloric, hydrobromic, oxalic, succinic, or benzenesulfonic acid, among others. Solvates of such addition salts are also included, for example, hydrated forms.

Furthermore, any crystalline form of dabigatran etexilate or a pharmaceutically acceptable salt thereof is included within the scope of the present invention.

For example, the crystalline forms I or II of dabigatran etexilate mesylate, as disclosed in WO2005/028468-A1, may be used.

In a preferred embodiment, dabigatran etexilate is in the form of salt with methanesulfonic acid, commonly known as dabigatran etexilate mesylate (DEM), or a solvate thereof, for example a hydrate thereof. Preferably, is in the form of anhydrous dabigatran etexilate mesylate.

In a particularly preferred embodiment, dabigatran etexilate is in the form of dabigatran etexilate mesylate crystalline form I, as described in WO2005/028468-A1.

Dabigatran etexilate or a pharmaceutically acceptable salt thereof, such as dabigatran etexilate mesylate, may be used in any particle size for preparing the pharmaceutical composition according to the present invention. For example, the active ingredient may typically have a particle size distribution wherein D₉₀ is not more than 25 µm, preferably wherein D₉₀ is not more than 10 µm. Said particle size distribution may be determined, for example, by laser diffraction (Malvern 2000).

Along the present description, when reference is made to the "active substance" in the formulation, it means the actual form used to prepare the formulation, either dabigatran etexilate or a pharmaceutically acceptable salt thereof, for example, dabigatran etexilate mesylate, irrespective that, pharmacologically, it is a prodrug and the real *in vivo* active agent might be dabigatran.

In the stated weight ratio between the pharmaceutically acceptable organic acid and the active substance, which is comprised between 0.15:1 and 0.40:1 according to the present invention, the weight of the active substance used in the calculation of said ratio is always the equivalent weight of dabigatran etexilate mesylate, even if dabigatran is used in the form of free base or in the form of another salt. Said equivalent weight of dabigatran etexilate mesylate is easily calculated from the respective molecular weights of the actual active substance and DEM.

The preparation of dabigatran etexilate, and pharmaceutically acceptable salts thereof is disclosed in the literature, for example, in WO98/37075-A1, WO03/074056-A1, WO2005/028468-A1, WO2006/000353-A1 or in Hauel et al., J. Med. Chem., 2002, 45, 1757.

Dabigatran etexilate, as well as pharmaceutically acceptable salts thereof and, in particular, dabigatran etexilate mesylate are also commercially available from several sources.

### Pharmaceutical composition

### The core

The core of the pharmaceutical composition according to the present invention comprises a pharmaceutically acceptable organic acid.

Typically, this organic acid has the function of providing an acid microenvironment after the dissolution of the composition, in order to maximise the dissolution and the absorption of the active ingredient.

The pharmaceutically acceptable organic acid used in the core of the composition has preferably a pKₐ value comprised between about 2 and about 5, preferably between 2 and 4.

Some pharmaceutically acceptable organic acids suitable to be used in the core of the composition are, for example, tartaric acid, fumaric acid, succinic acid, citric acid, malic acid, glutamic acid and aspartic acid. Said organic acids are meant to include any possible isomeric form thereof and also the hydrates and acid salts thereof.

In a preferred embodiment of the invention, the pharmaceutically acceptable organic acid in the core is tartaric acid.

Optionally, the core may also contain one or more pharmaceutically acceptable excipients.

Generally, the weight content of the pharmaceutically acceptable organic acid in the core is at least 80 wt%, preferably at least 85 wt%, more preferably at least 90 wt%, still more preferably at least 95 wt%, and still more preferably the core contains about 100 wt% of the pharmaceutically acceptable organic acid, wherein the percentages are based on the total weight of the core. Thus, in an embodiment of the invention, the core consists of pharmaceutically acceptable organic acid, preferably, the core consists of tartaric acid.

Generally, the core of the composition is formed by particles substantially spherical, having, at least 75% of the particles, a size in the range 0.4-1 mm, preferably 0.5-0.9 mm, and more preferably 0.6-0.8 mm. The size of the core beads may be determined, for example, by sieving.

When the core contains a pharmaceutically acceptable excipient, besides the pharmaceutically acceptable organic acid, it is generally selected from binding agents, diluents and the mixture thereof.

A description of the common pharmaceutically acceptable excipients used for the preparation the pharmaceutical compositions of the present invention can be found in the book R.C. Rowe, P.J. Sheskey & M.E. Quinn, Handbook of Pharmaceutical Excipients, 6th Edition, Pharmaceutical Press, 2009.

Suitable binding agents include acacia gum, agar, carbomers, copovidone, starch, hydroxypropyl cellulose, hypromellose, hydroxyethyl cellulose, hydroxyethylmethyl cellulose, lactose, maltose, methylcellulose, carmellose sodium povidone or sucrose, for example, and the mixture thereof.

Suitable diluents include cellulose, cellulose acetate, fructose, lactose, maltose, mannitol, microcrystalline cellulose, sorbitol, starch, sucrose, talc, trehalose and xylitol, among others, and the mixture thereof.

The cores may be prepared according to methods known in the art. For example, the preparation of tartaric acid cores is disclosed in WO03/074056-A1, WO2009/118321, WO2009/118322-A1 or in WO2010/007016-A1 by methods involving coating tartaric acid particles with a solution of tartaric acid and a binder, optionally with layered sprinkling of tartaric acid powder. The preparation of tartaric acid cores is also disclosed in WO2012/001156-A2 by applying a coating solution comprising tartaric acid and, optionally, a binding agent onto neutral commercial pellets, beadlets or spheres, for example, made of sugar, microcrystalline cellulose, mannitol or starch. Analogous cores comprising other organic acids may be prepared using analogous processes to those disclosed in the prior art for tartaric acid cores.

In a preferred embodiment, the cores consist essentially of the pharmaceutically acceptable organic acid, i.e., the content of organic acid in the cores is substantially the 100%.

The cores according to this embodiment, i.e., comprising substantially the 100% of organic acid, may be prepared using known methods in the pharmaceutical field, for example, by extrusion/spheronization, intensive mixing and granulation followed by size reduction.

Furthermore, some pellets made of an organic acid are also commercially available. For example, tartaric acid pellets are commercially available and may be used as cores. These tartaric acid pellets are spherical particles made of 100% tartaric acid and are commercially available, for example, from the companies Pharmatrans Sanaq AG or IPC Process-Center GmbH & Co., KG.

In a preferred embodiment, tartaric acid pellets are used as 100% tartaric acid cores, without any further coating over the pellets.

The weight content of the core, based on the total weight of the composition, is generally comprised between about 10 wt% and about 25 wt%, preferably comprised between 15 wt% and 20 wt%, more preferably comprised between 17 wt% and 18 wt% and still more preferably is about 18 wt%.

### The isolating layer

The composition may optionally have an isolating layer covering the core which has the function of separating the active substance from the organic acid in the core to avoid their interaction, in order to prevent the possible hydrolysis of dabigatran etexilate.

In an embodiment of the invention, the composition has an isolating layer between the organic acid core and the active substance layer, and the composition comprises:
a) a core comprising a pharmaceutically acceptable organic acid;
b) optionally, an isolating layer comprising a pharmaceutically acceptable polymer; and
c) an active substance layer comprising dabigatran etexilate or a pharmaceutically acceptable salt thereof, preferably dabigatran etexilate mesylate, and a binder;
wherein weight ratio between the pharmaceutically acceptable organic acid and the active substance is comprised between 0.15:1 and 0.40:1, and wherein the weight of the active substance in said ratio is expressed as equivalent weight of dabigatran etexilate mesylate.

In a preferred embodiment of the invention, the pharmaceutical composition does not have an isolating layer between the organic acid core and the active substance layer, and the composition comprises:
a) a core comprising a pharmaceutically acceptable organic acid; and
b) an active substance layer over the core comprising dabigatran etexilate or a pharmaceutically acceptable salt thereof, preferably dabigatran etexilate mesylate, and a binder;
wherein weight ratio between the pharmaceutically acceptable organic acid and the active substance is comprised between 0.15:1 and 0.40:1, and wherein the weight of the active substance in said ratio is expressed as equivalent weight of dabigatran etexilate mesylate.

It was found that, probably due to the smaller amount of organic acid in the composition, the stability was acceptable even without the presence of the isolating layer.

In another preferred embodiment of the invention, the composition has an isolating layer between the organic acid core and the active substance layer, and the composition comprises:
d) a core comprising a pharmaceutically acceptable organic acid;
e) an isolating layer comprising a pharmaceutically acceptable polymer; and
f) an active substance layer comprising dabigatran etexilate or a pharmaceutically acceptable salt thereof, preferably dabigatran etexilate mesylate, and a binder;
wherein weight ratio between the pharmaceutically acceptable organic acid and the active substance is comprised between 0.15:1 and 0.40:1, and wherein the weight of the active substance in said ratio is expressed as equivalent weight of dabigatran etexilate mesylate.

Said isolating layer, if present, comprises a suitable pharmaceutically acceptable polymer.

Said pharmaceutically acceptable polymers are typically coating or film-forming agents, as are well known for the skilled person in pharmaceutical formulation.

Suitable pharmaceutically acceptable polymers to be used in the isolating layer are acacia, carboxymethyl cellulose (CMC), carrageenan, cellulose acetate phthalate (CAP), chitosan, copovidone, ethyl cellulose (EC), guar gum, hydroxyethyl cellulose (HEC), hydroxyethylmethyl cellulose (HEMC), hydroxypropyl cellulose (HPC), hydroxypropyl methyl cellulose (hypromellose, HPMC), hydroxypropyl methyl cellulose phthalate (HPMCP), methylcellulose (MC), polyethylene oxide, polymethacrylates (including aminoalkyl methacrylate copolymers, butylated methacrylate copolymers, methacrylic acid-ethyl acrylate copolymers, methacrylic acid-methyl methacrylate copolymers, for example), polyvinyl acetate phthalate (PVAP), polyvinyl alcohol (PVA), polyvinylpyrrolidone (PVP), and xanthan gum among others, and the mixture thereof.

In a preferred embodiment of the invention, said pharmaceutically acceptable polymer is a water-soluble polymer.

Water-soluble polymers, within the framework of the present invention, mean those polymers which are suitable for immediate-release coatings, as are well-known to the skilled person in pharmaceutical formulation, and typically include polymers that are classified as sparingly soluble, soluble, very soluble or readily soluble in water.

Among the water-soluble polymers suitable to be used in the isolating layer are acacia, carboxymethyl cellulose (CMC), carrageenan, chitosan, guar gum, hydroxyethyl cellulose (HEC), hydroxypropyl cellulose (HPC), hydroxypropyl methyl cellulose (hypromellose, HPMC), polyvinylpyrrolidone (PVP), polyvinyl alcohol (PVA), copovidone and xanthan gum. Other suitable water-soluble polymers are aminoalkyl methacrylate copolymers which are readily soluble in aqueous media at low pH. Thus, in one embodiment of the invention, the water-soluble polymer is selected from the group consisting of acacia, aminoalkyl methacrylate copolymers, carboxymethyl cellulose (CMC), carrageenan, chitosan, copovidone, guar gum, hydroxyethyl cellulose (HEC), hydroxypropyl cellulose (HPC), hydroxypropyl methyl cellulose (hypromellose, HPMC), polyvinyl alcohol (PVA), polyvinylpyrrolidone (PVP), xanthan gum and the mixture thereof.

Preferably, the water-soluble polymer in the isolating layer is hydroxypropyl cellulose.

As is well-known in the field of pharmaceutical technology, hydroxypropyl cellulose is available in different grades, namely, in different molecular weights which give rise to different solution viscosities. In a preferred embodiment, the isolating layer comprises HPC having low viscosity, comprised between about 6.0 and about 10.0 mPa•s (corresponding to the viscosity of a 2 wt% aqueous solution at 20° C), which is available, for example, from the company Nisso as HPC-L, and has a molecular weight of about 140,000.

Optionally, the isolating layer also contains other excipients as formulation aids, such as anti-tacking agents and/or plasticizers. An anti-tacking agent is a substance able to reduce or prevent adhesion between particles. The anti-tacking agent can be selected, for example, from calcium silicate, magnesium silicate, colloidal silicon dioxide magnesium stearate, calcium stearate, magnesium oxide, talc and the mixture thereof. Preferably, the anti-tacking agent is talc. Plasticizers can be added in the isolating layer, for example, to increase the flexibility of the coating. Suitable plasticizers are polyethylene glycol, propylene glycol, diethyl phthalate or triethyl citrate, for example, among others.

In a preferred embodiment the isolating layer comprises a pharmaceutically acceptable polymer, preferably a pharmaceutically acceptable water-soluble polymer, and more preferably hydroxypropyl cellulose (HPC), and an anti-tacking agent, preferably talc, in a weight ratio polymer:anti-tacking agent comprised between 2:1 and 1:2, preferably comprised between 1.5:1 and 1:1.5, more preferably comprised between 1.2:1 and 1:1.2, still more preferably comprised between 1.1:1 and 1:1.1 and still more preferably is about 1:1.

The weight content of the isolating layer, based on the total weight of the composition, is comprised between 0.1 wt% and 4 wt%, preferably between 0.2 wt% and 2 wt%, more preferably comprised between 0.3 wt% and 1.5 wt%, still more preferably comprised between 0.4 wt% and 1.2 wt%, and still more preferably comprised between 0.5 wt% and 1 wt%.

### The active substance layer

The active substance layer covers the isolating layer, if present, or covers the organic acid core when there is no isolating layer, and comprises the active substance, i.e., dabigatran etexilate or a pharmaceutically acceptable salt thereof as defined above, and a binder.

The amount of the active substance, based on the total weight of this layer, is generally comprised between about 60 wt% and about 85 wt%, preferably comprised between 65 wt% and 80 wt%, and more preferably comprised between 70 wt% and 75 wt%, expressed as weight percentage.

The amount of the active substance, based on the total weight of the composition, is generally comprised between 40 wt% and 80 wt%, preferably comprised between 50 wt% and 70 wt%, and more preferably comprised between 55 wt% and 65 wt%, expressed as weight percentage.

Suitable binding agents include, for example, acacia gum, agar, carbomers, copovidone, hydroxypropyl cellulose, carmellose sodium, polyvinyl alcohol, hypromellose, hydroxyethyl cellulose, hydroxyethylmethyl cellulose, methylcellulose and povidone, among others, and the mixture thereof. Preferably, the binding agent is selected from the group consisting of hydroxypropyl cellulose, hypromellose, povidone, copovidone, and the mixture thereof; and more preferably, the binding agent is hydroxypropyl cellulose.

In a preferred embodiment, the binder is hydroxypropyl cellulose having low viscosity, preferably comprised between about 2.0 and about 10.0 mPa•s (expressed as the viscosity of a 2 wt% aqueous solution at 20° C), more preferably comprised between about 2.0 and about 5.9 mPa•s, and still more preferably comprised between about 2.0 and about 2.9 mPa•s. These grades of low-viscosity hydroxypropyl cellulose are commercially available, for example, from the company Nisso as the grades HPC-L, HPC-SL and HPC-SSL, which have a molecular weight of about 140,000, 100,000 and 40,000, respectively. In a particularly preferred embodiment, the hydroxypropyl cellulose used in the active substance layer is a very low viscosity hydroxypropyl cellulose, such as HPC-SSL (Nisso) having a viscosity in the range 2.0-2.9 mPa•s.

The amount of binding agent in this layer is generally comprised between about 10 wt% and about 30 wt%, preferably comprised between 12 wt% and 20 wt%, expressed as weight percentage based on the total weight of the layer.

Optionally, the active substance layer also contains an anti-tacking agent. The anti-tacking agent can be selected, for example, from calcium silicate, magnesium silicate, colloidal silicon dioxide magnesium stearate, calcium stearate, magnesium oxide, talc and the mixture thereof. Preferably, the anti-tacking agent is talc.

When the active substance layer comprises an anti-tacking agent, it is generally in an amount comprised between about 5 wt% and about 20 wt%, preferably comprised between 10 wt% and 15 wt%, expressed as weight percentage based on the total weight of the layer.

The weight content of the active substance layer, based on the total weight of the composition, is generally comprised between about 75 wt% and about 90 wt%, preferably comprised between 80 wt% and 85 wt%.

In a preferred embodiment the active substance layer comprises a binding agent, preferably hydroxypropyl cellulose (HPC) and an anti-tacking agent, preferably talc in a weight ratio binder:anti-tacking agent comprised between 2:1 and 0.75:1, preferably comprised between 1.5:1 and 1:1, more preferably comprised between 1.4:1 and 1.1:1, and still more preferably is about 1.3:1.

The present pharmaceutical composition is wherein the weight ratio between the pharmaceutically acceptable organic acid in the core and the active substance is comprised between 0.15:1 and 0.40:1. Any weight ratio comprised within this range and as well as any sub-ranges comprised within this range are included into the scope of the present invention. Included sub-ranges are, for example, those comprised between any one of (0.15:1), (0.16:1), (0.17:1), (0.18:1), (0.19:1), (0.20:1), (0.21:1), (0.22:1), (0.23:1), (0.24:1), (0.25:1), (0.26:1), (0.27:1), (0.28:1), (0.29:1) or (0.30:1), as lower end of the range, and any one of (0.30:1), (0.31:1), (0.32:1), (0.33:1), (0.34:1), (0.35:1), (0.36:1), (0.37:1), (0.38:1), (0.39:1) or (0.40:1), as upper end of the range. In a preferred embodiment, the weight ratio between pharmaceutically acceptable the organic acid in the core and the active substance is comprised between 0.20:1 and 0.38:1, more preferably comprised between 0.25:1 and 0.35:1, and more preferably is about 0.30:1. In all the above stated ratios between the organic acid and the active substance, the weight of active substance is expressed as equivalent weight of dabigatran etexilate mesylate.

The amount of organic acid, based on the total weight of the composition, is generally comprised between 10 wt% and 25 wt%, preferably between 15 wt% and 20 wt%, more preferably between 17 wt% and 18 wt%, and still more preferably is about 18 wt%.

The total weight content of additional excipients in the pharmaceutical composition, excluding the pharmaceutically acceptable organic acid, i.e., the polymer in the isolating layer, the binder in the active substance layer, and other optional additional additives, such as plasticizers and anti-tacking agents, is generally comprised between 2 wt% and 40 wt%, preferably comprised between 10 wt% and 30 wt%, more preferably comprised between 15 wt% and 30 wt%, and still more preferably comprised between 20 wt% and 25 wt% based on the total weight of the composition.

The pharmaceutical composition of the invention as disclosed herein is typically in the form of pellets, or beadlets, i.e., roundish, roughly spherical particles having a size generally from about 0.5 mm to about 4.0 mm, preferably from about 0.5 mm to about 2.5 mm, preferably from about 1.0 mm to about 2.0 mm, expressed as D₉₀ particle size distribution. The particle size is determined by the size of the organic acid core, plus the thickness of the coatings.

Optionally, the pharmaceutical composition may have an additional overcoat (or finishing layer) applied onto the active substance layer. This overcoat generally comprises hypromellose, povidone, hydroxypropyl cellulose (HPC), or the mixture thereof. Preferably comprises hydroxypropyl cellulose.

Optionally, said overcoat layer may also contain an anti-tacking agent. The anti-tacking agent can be selected, for example, from calcium silicate, magnesium silicate, colloidal silicon dioxide magnesium stearate, calcium stearate, magnesium oxide, talc and the mixture thereof. Preferably, the anti-tacking agent is talc.

Preferably, when there is a finishing layer, it comprises a mixture of the polymer and the anti-tacking agent, preferably a mixture of HPC and talc. Preferably, the weight ratio HPC:talc is comprised between 2:1 and 1:2, more preferably comprised between 1.5:1 and 1:1.5, still more preferably comprised between 1.2:1 and 1:1.2, still more preferably comprised between 1.1:1 and 1:1.1 and still more preferably is about 1:1.

When there is a finishing layer, the weight content of this layer is from about 3 wt% to about 8 wt% based on the total weight of the formulation (before applying said finishing layer).

When there is an additional finishing layer, the percentages stated along the present description that refer to "the total weight of the composition" are calculated without including the weight of the finishing layer, i.e., they refer to the weight of the composition before applying the finishing layer.

In a preferred embodiment, the pharmaceutical composition comprises:
a) a core comprising a pharmaceutically acceptable organic acid, and preferably the core consists essentially of a pharmaceutically acceptable organic acid; wherein the organic acid is selected from the group consisting of tartaric acid, fumaric acid, succinic acid, citric acid, malic acid, glutamic acid and aspartic acid, preferably the pharmaceutically acceptable organic acid is tartaric acid;
b) optionally, an isolating layer comprising a water-soluble polymer and optionally an anti-tacking agent and/or a plasticiser, wherein the water-soluble polymer is preferably selected from the group consisting of acacia, aminoalkyl methacrylate copolymers, carboxymethyl cellulose (CMC), carrageenan, chitosan, copovidone, guar gum, hydroxyethyl cellulose (HEC), hydroxypropyl cellulose (HPC), hydroxypropyl methyl cellulose (hypromellose, HPMC), polyvinyl alcohol (PVA), polyvinylpyrrolidone (PVP), xanthan gum and the mixture thereof, and more preferably the water-soluble polymer is hydroxypropyl cellulose (HPC); and wherein the anti-tacking agent is preferably selected from the group consisting of calcium silicate, magnesium silicate, colloidal silicon dioxide magnesium stearate, calcium stearate, magnesium oxide, talc and the mixture thereof, and more preferably the anti-tacking agent is talc;
c) an active substance layer comprising dabigatran etexilate mesylate, a binder and optionally an anti-tacking agent, wherein the binder is preferably selected from the group consisting of hydroxypropyl cellulose, hypromellose, povidone, and copovidone, more preferably, the binder is hydroxypropyl cellulose; and wherein the anti-tacking agent is preferably selected from the group consisting of calcium silicate, magnesium silicate, colloidal silicon dioxide magnesium stearate, calcium stearate, magnesium oxide, talc and the mixture thereof, and more preferably the anti-tacking agent is talc;
wherein the weight ratio between pharmaceutically acceptable the organic acid and the active substance is comprised between 0.15:1 and 0.40:1, preferably comprised between 0.20:1 and 0.38:1, more preferably comprised between 0.25:1 and 0.35:1 and still more preferably is about 0.30:1, wherein the weight of active substance in said ratio is expressed as equivalent weight of dabigatran etexilate mesylate.

In a particularly preferred embodiment, the pharmaceutical composition comprises:
a) a core consisting of tartaric acid;
b) an isolating layer comprising hydroxypropyl cellulose (HPC) and talc, wherein the weight ratio HPC:talc is preferably comprised between 2:1 and 1:2, more preferably comprised between 1.5:1 and 1:1.5, still more preferably comprised between 1.2:1 and 1:1.2, still more preferably comprised between 1.1:1 and 1:1.1 and still more preferably is about 1:1 ; and
c) an active substance layer comprising dabigatran etexilate mesylate, hydroxypropyl cellulose (HPC) and talc, wherein the amount of dabigatran etexilate mesylate in this layer is comprised between 60 wt% and 85 wt%, preferably comprised between 65 wt% and 80 wt%, and more preferably comprised between 70 wt% and 75 wt%, based on the total weight of the layer, and wherein the weight ratio HPC:talc is preferably comprised between 2:1 and 0.75:1, more preferably comprised between 1.5:1 and 1:1, still more preferably comprised between 1.4:1 and 1.1:1, and still more preferably is about 1.3:1; and
wherein the weight ratio between tartaric acid and dabigatran etexilate mesylate is comprised between 0.15:1 and 0.40:1, preferably comprised between 0.20:1 and 0.38:1, more preferably comprised between 0.25:1 and 0.35:1, and more preferably is about 0.30:1;
wherein, preferably, HPC in the isolating layer has a viscosity comprised between about 6.0 and about 10.0 mPa•s (corresponding to the viscosity of a 2 wt% aqueous solution at 20° C); and, preferably, HPC in the active substance layer has viscosity comprised between about 2.0 and about 10.0 mPa•s (expressed as the viscosity of a 2 wt% aqueous solution at 20° C), more preferably comprised between about 2.0 and about 5.9 mPa•s, and still more preferably comprised between about 2.0 and about 2.9 mPa•s:
and wherein the total weight content HPC and talc in the composition is preferably comprised between 2 wt% and 40 wt%, more preferably comprised between 10 wt% and 30 wt%, still more preferably comprised between 15 wt% and 30 wt%, and still more preferably comprised between 20 wt% and 25 wt%, based on the total weight of the composition.

In other particularly preferred embodiment, the pharmaceutical composition comprises, as described above, a further overcoat layer.

### Method of preparation

The method of preparation of the pharmaceutical composition according to the invention comprises the following steps:
i) optionally, coating the pharmaceutically acceptable organic acid cores with an isolating coating solution or suspension comprising the pharmaceutically acceptable polymer and, optionally, an additional pharmaceutically acceptable excipient, dissolved or suspended in a solvent; and
ii) coating the pharmaceutically acceptable organic acid cores or the pellets obtained in step i) with an active substance coating solution or suspension comprising dabigatran etexilate or a pharmaceutically acceptable salt thereof, a binder and, optionally, an additional pharmaceutically acceptable excipient, dissolved or suspended in a solvent.

In a preferred embodiment, the method comprises the following steps:
i) coating the pharmaceutically acceptable organic acid cores with an isolating coating solution or suspension comprising the pharmaceutically acceptable polymer and, optionally, an additional pharmaceutically acceptable excipient, dissolved or suspended in a solvent; and
ii) coating the pellets obtained in step i) with an active substance coating solution or suspension comprising dabigatran etexilate or a pharmaceutically acceptable salt thereof, a binder and, optionally, an additional pharmaceutically acceptable excipient, dissolved or suspended in a solvent.

The coating steps may be performed according to processes well-known in the art. For example, a fluidized-bed apparatus can be used, wherein the coating solution or suspension is applied continuously from a spray nozzle, while the particles being coated are kept in motion and dried with an air stream.

The characteristics and preferred embodiments of the cores, the pharmaceutically acceptable polymer and optional additional pharmaceutically acceptable excipient of step i) are as disclosed above in the core and isolating layer sections.

The characteristics and preferred embodiments of dabigatran etexilate or a pharmaceutically acceptable salt thereof, the binder and additional pharmaceutically acceptable excipient of step ii) are as disclosed above in the dabigatran and active substance layer sections.

Once all the coating solution/suspension has been sprayed, the coated pellets are kept in the fluidized bed and dried under the action of the air stream, so the solvent is removed and is not present in the final product.

The solvent used for preparing the coating solutions or suspensions may be water, a suitable organic solvent or the mixture thereof. Preferably, a suitable organic solvent is used and water is preferably avoided, especially for the preparation of the active substance layer due to the susceptibility of the active agent to hydrolysis.

Suitable organic solvents include, but are not limited to, methanol, ethanol, isopropanol, dimethylformamide, and acetone, for example. A preferred solvent is isopropanol.

The coating solutions/suspensions may be prepared following common procedures, typically involving the dissolution or dispersion of the solid ingredient(s) in the solvent, under stirring, in a suitable mixing apparatus, for example, in a high-shear homogenizer and/ or low speed mixer.

For example, for preparing the coating solution or suspension of step i) typically the pharmaceutically acceptable polymer, e.g. HPC, is first gradually added, in portions, to the solvent (e.g. isopropanol) under stirring in a high-shear homogenizer until dissolution and then the additional pharmaceutically acceptable excipient, if present, preferably an anti-tacking agent such as talc, is gradually added, in portions, under stirring until an homogeneous suspension is obtained. The solution/suspension is passed to the fluidized bed to be sprayed onto the acid cores. The solution/suspension is kept under stirring during the fluid bed coating process.

Analogously, for preparing the coating solution or suspension of step ii) typically the binder, e.g. HPC, is first gradually added to the solvent (e.g. isopropanol) under stirring in a high-shear homogenizer until complete dissolution; next, the additional pharmaceutically acceptable excipient, if present, preferably an anti-tacking agent such as talc, is finally added, also gradually, in portions, under stirring, preferably maintaining the stirring for about 15-45 additional minutes until an homogeneous suspension is obtained, and then, the active ingredient is gradually added, in portions, also under continuous stirring, preferably maintaining the stirring for about 15-45 additional minutes after all the active has been added. Preferably, the solution or suspension of step ii) is prepared and kept at a temperature not exceeding 30° C, preferably comprised between 5° C and 30° C, more preferably comprised between 10° C and 20° C. The obtained solution/suspension is passed to the fluidized bed to be sprayed onto the isolated pellets obtained in step i).

In case that an additional finishing layer is present, the method includes an additional step:
iii) coating the pellets obtained in step ii) with a finishing layer.

If present, the finishing layer is prepared analogously as disclosed for steps i) and ii).

The coating processes are well known in the pharmaceutical technology field and are disclosed in reference books such as Aulton's Pharmaceutics. The design and manufacture of medicines, M.E. Aulton and K.M.G. Taylor, editors, Churchill Livingstone Elsevier, Fourth Edition, 2013; or in the book Remington Essentials of Pharmaceutics, L. Felton, editor, Pharmaceutical Press, 2013; or in the book Pharmaceutics. Basic principles and application to pharmacy practice. A.K. Dash, S. Singh, and J. Tolman, editors, Academic Press, Elsevier, 2014.

### Pharmaceutical dosage form

Another aspect of the invention is a pharmaceutical dosage form comprising the pharmaceutical composition of the invention.

In a preferred embodiment of the invention, the pharmaceutical dosage form is a capsule.

Preferably hard capsules are used, typically gelatin or hypromellose capsules. Hypromellose capsules are generally preferred due to their low moisture content, to avoid hydrolysis of the active ingredient and thus increase the stability of the composition.

Typically, the capsule is filled with the pharmaceutical composition, in the form of multi-layered pellets as described above, in an amount suitable to provide a therapeutically effective amount of dabigatran etexilate, or a pharmaceutically acceptable salt thereof.

Preferably each dosage form, i.e. each capsule, contains an amount of dabigatran etexilate or a pharmaceutically acceptable salt thereof comprised between about 50 mg and 200 mg, preferably comprised between about 75 mg and 150 mg, expressed as the equivalent amount of dabigatran etexilate free base.

In a preferred embodiment, each dosage form contains about 75 mg of dabigatran etexilate.

In another preferred embodiment, each dosage form contains about 110 mg of dabigatran etexilate.

In another preferred embodiment, each dosage form contains about 110 mg of dabigatran etexilate.

### Use of the pharmaceutical composition

The oral pharmaceutical composition according to the present invention, comprising dabigatran etexilate or a pharmaceutically acceptable salt thereof, preferably the mesylate salt, has proved to provide a good bioavailability, and is bioequivalent to the commercial medicine, i.e., to Pradaxa® dabigatran etexilate mesylate. Therefore, it is suitable for the same therapeutic uses as the reference medicine.

Furthermore, the present composition has advantages over the commercial composition due to the reduced amount of excipients contained in the formulation, particularly, the lower amount of acid. Indeed, it was surprisingly found that the present composition was bioequivalent to Pradaxa® despite comprising about 60-80% less of tartaric acid. Therefore, the present composition is advantageous in terms of safety, as the possible adverse events related to the excipients are minimized, in particular, the expected erosive gastric effect due to the acid.

On the other hand, the present invention may also be advantageous in terms of patient compliance because, as less weight of composition is required for a given strength of active ingredient, the size of the dosage forms may be reduced, in particular, the size of the capsules to be administered.

Therefore, another aspect of the invention is the pharmaceutical composition according to the invention, or the pharmaceutical dosage form comprising it for use in therapy. In particular, the composition is suitable for use in the treatment and/or prevention of blood coagulation, thromboembolic events, and stroke. Some thromboembolic events suitable to be treated/prevented with the composition of the invention are, for example, deep vein thrombosis, venous thromboembolism, pulmonary embolism, and systemic embolism.

### Examples

### Example 1A Preparation of dabigatran etexilate mesylate capsules according to the invention

150 mg strength dabigatran etexilate capsules were prepared, filled with the multilayered pellets according to the present invention using the ingredients shown in Table II. The ratio tartaric acid:active substance (DEM) in this example is 0.30:1.

**TABLE II**

| *Layer* | *Ingredient* | *% layer* | *% composition* | *mg*/*capsule* |
|---|---|---|---|---|
| Core | TAP 700 pellets | 100.0 | 17.77 | 51.89 |
| Isolating layer | HPC-L | 50.0 | 0.45 | 1.30 |
| | Talc | 50.0 | 0.45 | 1.30 |
| Active substance layer | DEM | 72.8 | 59.23 | 172.96 |
| | HPC-SSL | 14.6 | 11.86 | 34.64 |
| | Talc | 12.6 | 10.24 | 29.91 |
| Total | | | 100.00 | 292.00 |

For the tartaric acid core, 100% tartaric acid pellets TAP 700 (Pharmatrans Sanaq AG or IPC Process-Center GmbH & Co., KG.) were used (having particle size distribution such that at least 75% of the pellets have a diameter of 600-800 µm).

For preparing the isolating layer coating suspension, hydroxypropyl cellulose (HPC-L, Nisso, viscosity 6.0-10 mPa•s 20°C/2%aq) was slowly added to stirred isopropanol in a high-shear mixer until completely dissolved (the amount of isopropanol used was about 21 times the weight of HPC). Talc was slowly added to the solution and mixed for at least 30 minutes. The suspension was sprayed onto the starting tartaric acid pellets in a fluidized bed reactor. After spraying the suspension, the pellets were dried in the same fluidized bed apparatus.

The active substance layer suspension was prepared at about 14° C. Hydroxypropyl cellulose (HPC-SSL, Nisso, viscosity 2.0-2.9 mPa•s 20°C/2%aq) was first slowly added to stirred isopropanol in a high-shear mixer until completely dissolved (the amount of isopropanol used was about 27 times the weight of HPC). Then, talc was first added followed by dabigatran etexilate mesylate (DEM), both slowly, in small portions, and the mixture was stirred for about 30 minutes. This suspension was sprayed onto the isolated pellets of the previous step in a fluidized bed reactor. After spraying the suspension, the pellets were dried in the same fluidized bed apparatus.

292.00 mg of the obtained composition were filled into HPMC capsules. In this case, capsules size "0" were selected, i.e., the same size as Pradaxa®, as they were to be used in comparative bioavailability studies with the reference medicament and, thus, the same size of the compared dosage forms was required. However, due to the reduced amount of the final composition (292 mg) smaller capsules (size 1) might be used. Each capsule contained 172.96 mg of dabigatran etexilate mesylate, equivalent to 150 mg of dabigatran etexilate.

Analogously, 75 mg and 110 mg strength dabigatran etexilate capsules were prepared. The ingredients per capsule for each strength is shown in Table III:

**TABLE III**

| | | *mg*/*capsule* | | |
|---|---|---|---|---|
| *Layer* | *Ingredient* | *75 mg* | *110 mg* | *150 mg* |
| Core | TAP 700 pellets | 25.95 | 38.05 | 51.89 |
| Isolating layer | HPC-L | 0.65 | 0.95 | 1.30 |
| | Talc | 0.65 | 0.95 | 1.30 |
| Active substance layer | DEM | 86.48 | 126.84 | 172.96 |
| | HPC-SSL | 17.32 | 25.40 | 34.64 |
| | Talc | 14.95 | 21.94 | 29.91 |
| Total | | 146 | 214 | 292 |

The 75 mg and 110 mg strength compositions were filled into size 3 and 2 capsules, respectively.

### Example 1B Preparation of dabigatran etexilate mesylate capsules according to the invention

Further 150 mg strength dabigatran etexilate capsules were prepared, filled with the multilayered pellets according to the present invention which were prepared using the ingredients shown in Table IV. The ratio fumaric acid:active substance (DEM) in this example is 0.17:1.

**TABLE IV**

| *Layer* | *Ingredient* | *% layer* | *% composition* | *mg*/*capsule* |
|---|---|---|---|---|
| Core | Fumaric acid granules | 100.0 | 10.91 | 29.40 |
| Isolating layer | Surelease® 25% (w/w) | 100.0 | 0.96 | 2.59 |
| Active substance layer | DEM | 72.82 | 64.18 | 172.96 |
| | HPC-SSL | 14.58 | 12.85 | 34.64 |
| | Talc | 12.60 | 11.10 | 29.91 |
| Total | | | 100.00 | 269.50 |

For the fumaric acid cores, fumaric acid granules produced by extrusión/spheronization method, were used. The final size of the cores is around 0.7mm (tested by siving).

For preparing the isolating layer, an aqueous ethylcellulose dispersion commercially available from Colorcon (Surelease®) was used, which is supplied as a 25% (w/w) solids dispersion, and was diluted with water to 15% (w/w) solids before use. Before dilution, the container of Surelease® was agitated to ensure homogenization of solids in the dispersion, and then the dispersion was diluted by adding two parts of purified water to three parts of Surelease® and stirred with a low shear mixer for approximately 15 min.

The suspension was sprayed onto the starting fumaric acid cores in a fluidized bed reactor. After spraying the suspension, the cores were dried in the same fluidized bed apparatus.

The active substance layer suspension was prepared in the same way as disclosed in Example 1A and was sprayed onto the isolated pellets of the previous step in a fluidized bed reactor. After spraying the suspension, the pellets were dried in the same fluidized bed apparatus.

269.50 mg of the obtained composition were filled into HPMC capsules. Analogously, 75 mg and 110 mg strength dabigatran etexilate capsules were prepared using proportional amount of pellets.

### Example 1C Preparation of dabigatran etexilate mesylate capsules according to the invention

Further 150 mg strength dabigatran etexilate capsules were prepared, filled with the multilayered pellets according to the present invention which were prepared using the ingredients shown in Table V. The ratio tartaric acid:active substance (DEM) in this example is 0.38:1.

**TABLE V**

| *Layer* | *Ingredient* | *% layer* | *% composition* | *mg*/*capsule* |
|---|---|---|---|---|
| Core | TAP 500 pellets | 100.0 | 22:64 | 66.11 |
| Isolating layer | PVP K-30 | 100.0 | 2.27 | 6.62 |
| Active substance layer | DEM | 78.88 | 59.23 | 172.96 |
| | HPC-SSL | 21.12 | 15.86 | 46.31 |
| Total | | | 100.00 | 292.00 |

For the tartaric acid core, 100% tartaric acid pellets TAP 500 (Pharmatrans Sanaq AG or IPC Process-Center GmbH & Co., KG.) were used.

For the isolating layer, polyvinylpyrrolidone (PVP) was used, namely, polyvinylpyrrolidone grade PVP K-30 (BASF). A solution of PVP K-30 in isopropanol was sprayed onto the starting tartaric acid pellets in a fluidized bed reactor. After spraying the solution, the pellets were dried in the same fluidized bed apparatus.

The active substance layer suspension was prepared analogously as disclosed in Example 1A, but without adding talc, and was sprayed onto the isolated pellets of the previous step in a fluidized bed reactor. After spraying the suspension, the pellets were dried in the same fluidized bed apparatus.

292.00 mg of the obtained composition were filled into HPMC capsules. Analogously, 75 mg and 110 mg strength dabigatran etexilate capsules were prepared using proportional amount of pellets.

### Example 2 Comparative in vitro dissolution test

The solubility of the 150 mg capsules prepared in Example 1A was compared with the solubility of 150 mg strength Pradaxa® capsules.

For the comparative dissolution test, a paddle method was used using 900 ml of hydrochloric acid-sodium chloride buffer (pH 2.0) at 37°C as dissolution medium at a rotation speed of 50 rpm.

The percentage of dabigatran etexilate mesylate dissolved at time points 10, 15, 20, 30, 45 and 60 is shown in Table VI below. The values shown are the average values of 6 units dissolution assays.

**TABLE VI**

| *Time (min)* | *% DEM dissolved* | |
|---|---|---|
| | *150 mg strength Example 1A* | *150 mg strength Pradaxa*® |
| 10 | 3.4 | 12.9 |
| 15 | 19.1 | 32.2 |
| 20 | 34.7 | 48.8 |
| 30 | 69.2 | 79.7 |
| 45 | 93.2 | 95.0 |
| 60 | 97.4 | 97.0 |

The results are represented in Figure 1.

Both formulations were completely dissolved after 60 minutes.

### Example 3 Comparative bioavailability

The bioavailability of the 150 mg dabigatran etexilate strength capsule of Example 1A, according to the present invention, was compared to the bioavailability of a commercial 150 mg strength of Pradaxa®.

The assessed parameters were Cₘₐₓ and AUC_{0-T}.

The results are shown in Table VII:

**TABLE VII**

| *Parameter* | *Example 1A* | *Pradaxa*® | Ratio (%) |
|---|---|---|---|
| Cₘₐₓ (ng/ml) | 116.77 | 114.62 | 101.88 |
| AUC_{0-T} (ng·h/ml) | 903.06 | 887.30 | 101.78 |

The data of Cₘₐₓ and AUC_{0-T} for both formulations (second and third columns of the Table) were calculated as the geometric LS mean of the data for all the subjects.

It was surprisingly found that the composition according to the present invention, having 0.30:1 tartaric acid:dabigatran etexilate mesylate ratio and, therefore, containing much less tartaric acid than the reference medicine (51.89 mg vs. 177.18 mg) was bioequivalent to Pradaxa®.

## Claims

1. Pharmaceutical composition for oral administration comprising:
a) a core comprising a pharmaceutically acceptable organic acid;
b) optionally, an isolating layer comprising a pharmaceutically acceptable polymer; and
c) an active substance layer comprising dabigatran etexilate or a pharmaceutically acceptable salt thereof, preferably dabigatran etexilate mesylate, and a binder;
wherein weight ratio between the pharmaceutically acceptable organic acid and the active substance is comprised between 0.15:1 and 0.40:1, and wherein the weight of the active substance in said ratio is expressed as equivalent weight of dabigatran etexilate mesylate.

2. The pharmaceutical composition of claim 1, wherein it comprises the isolating layer comprising a pharmaceutically acceptable polymer and the isolated layer is between the core and the active substance layer.

3. The pharmaceutical composition according to claims 1 or 2, wherein the pharmaceutically acceptable organic acid is selected from the group consisting of tartaric acid, fumaric acid, succinic acid, citric acid, malic acid, glutamic acid and aspartic acid, preferably is tartaric acid.

4. The pharmaceutical composition according to any one of claims 1 to 3, wherein the weight ratio between the pharmaceutically acceptable organic acid and the active substance is comprised between 0.20:1 and 0.38:1, preferably comprised between 0.25:1 and 0.35:1, more preferably is about 0.30:1.

5. The pharmaceutical composition according to any one claims 1 to 4, wherein the weight content of the pharmaceutically acceptable organic acid in the core is at least 80 wt% based on the total weight of the core, preferably the core consists of pharmaceutically acceptable organic acid.

6. The pharmaceutical composition according to any one of claims 1 to 5, wherein the pharmaceutically acceptable polymer in the isolating layer is a water-soluble polymer, preferably selected from the group consisting of acacia, aminoalkyl methacrylate copolymers, carboxymethyl cellulose (CMC), carrageenan, chitosan, copovidone, guar gum, hydroxyethyl cellulose (HEC), hydroxypropyl cellulose (HPC), hydroxypropyl methyl cellulose (hypromellose, HPMC), polyvinyl alcohol (PVA), polyvinylpyrrolidone (PVP), xanthan gum, and the mixture thereof.

7. The pharmaceutical composition according to claim 6, wherein the water-soluble polymer is hydroxypropyl cellulose, preferably, low viscosity hydroxypropyl cellulose of viscosity comprised between 6.0 and 10.0 mPa•s, expressed as the viscosity of a 2 wt% aqueous solution at 20° C.

8. The pharmaceutical composition according to any one of claims 1 to 7, wherein the isolating layer also comprises an anti-tacking agent, preferably talc, in a weight ratio polymer:anti-tacking agent comprised between 2:1 and 1:2.

9. The pharmaceutical composition according to any one of claims 1 to 8, wherein the weight content of the isolating layer is comprised between 0.1 wt% and 4 wt%, based on the total weight of the composition.

10. The pharmaceutical composition according to any one of claims 1 to 9, wherein the binder in the active substance layer is selected from the group consisting of hydroxypropyl cellulose, hypromellose, povidone, copovidone, and the mixture thereof; preferably is hydroxypropyl cellulose, more preferably is low viscosity hydroxypropyl cellulose of viscosity comprised between 2.0 and 10.0 mPa•s, expressed as the viscosity of a 2 wt% aqueous solution at 20° C.

11. The pharmaceutical composition according to any one of claims 1 to 10, wherein the active substance layer also comprises an anti-tacking agent, preferably talc, in an amount comprised between 5 wt% and 20 wt%, based on the total weight of the layer.

12. The pharmaceutical composition according to any one of claims 1 to 11, wherein the weight content of the active substance layer is comprised between 75 wt% and about 90 wt%, based on the total weight of the composition.

13. A method of preparation of the pharmaceutical composition according to any one of claims 1 to 12, wherein it comprises the following steps:
i) optionally, coating the pharmaceutically acceptable organic acid cores with an isolating coating solution or suspension comprising the pharmaceutically acceptable polymer and, optionally, an additional pharmaceutically acceptable excipient, dissolved or suspended in a solvent; and
ii) coating the pharmaceutically acceptable organic acid cores or the cores with the isolating coating obtained in step (i) with an active substance coating solution or suspension comprising dabigatran etexilate or a pharmaceutically acceptable salt thereof, a binder and, optionally, an additional pharmaceutically acceptable excipient, dissolved or suspended in a solvent.

14. A pharmaceutical dosage form comprising the pharmaceutical composition according to any one of claims 1 to 12, preferably in the form of a capsule.

15. The pharmaceutical composition according to any one of claims 1 to 12 or the pharmaceutical dosage form of claim 14 for use in therapy, preferably, for use in the treatment and/or prevention of blood coagulation, thromboembolic events and stroke.
